# EUROPEAN PATENT SPECIFICATION

(11) **EP 4 364 700 B1**
(45) Date of publication and mention of the grant of the patent: **12.11.2025**
(21) Application number: 23199901.2
(22) Date of filing: 26.09.2023
(51) Int. Cl.: A61F 2/46, A61B 17/15

(54) **DEVICE FOR IDENTIFYING THE CORRECT POSITIONING OF THE FEMORAL COMPONENT IN PROSTHETIC KNEE SURGERY**
VORRICHTUNG ZUR ERKENNUNG DER KORREKTEN POSITIONIERUNG DER FEMURKOMPONENTE IN DER KNIEGELENKSPROTHESE
DISPOSITIF POUR IDENTIFIER LE POSITIONNEMENT CORRECT DU COMPOSANT FÉMORAL DANS UNE CHIRURGIE DU GENOU PROTHÉTIQUE

(30) Priority: 07.11.2022 IT 202200022815
(43) Date of publication of application: 08.05.2024
(73) Proprietor: Adler Ortho S.p.a., 20032 Cormano (IT); Schiraldi, Marco, 12038 Savigliano CN (IT)
(72) Inventor: SCHIRALDI, Marco, 12038 Savigliano CN (IT)
(74) Representative: Modiano, Micaela Nadia

(56) References cited:
- EP-A1- 3 009 110
- US-A1- 2011 071 533
- US-A1- 2012 310 246
- US-B1- 6 645 215
- US-B2- 7 141 053

## Description

The present invention relates to a device for identifying the correct positioning of the femoral component in prosthetic knee surgery. More specifically, the invention relates to a device that makes it possible to identify the correct positioning of the femoral component in prosthetic knee surgery according to the flexion-extension axis of the patella, an axis that by convention is parallel to the tibiofemoral flexion-extension axis.

As is known, in prosthetic knee surgery, restoring a neutral alignment of the knee is considered one of the main characteristics of why knee surgery is successful, in order to obtain a neutral mechanical axis that passes from the center to the femoral head through the center of activation of the knee down to the center of the ankle.

The method generally used is mechanical alignment. It requires an initial femoral cut that needs to be perpendicular to the mechanical axis of the femur. The tibial resection then needs to be executed perpendicular to the mechanical axis of the tibia. The result is a knee that is generally aligned with a valgus deformity angle of 5 degrees.

The objective of the desired alignment is to create a uniform distribution of load on the new joint that is to be implanted, in order to ensure a satisfactory clinical outcome and an adequate lifetime of the implant.

Notwithstanding the above, the alignment of the knee is not a condition that is always sufficient to ensure the success of the implant for the patient.

This is also in consideration of the fact that the desired alignment often tends to alter the tension of the ligaments and so cause unwanted stresses and, in the end, pain for the patient when moving the knee.

Nowadays alternative methods of alignment have been devised (purely kinematic, restricted, reverse, functional) as solutions to the above-mentioned problems.

None of the provided methods highlights the importance of the rotatory component of the femur, which is still left to the intuition of the surgeon.

EP 3 009 110, US 2012/310246 and US 2011/071533 disclose devices and methods for fitting a knee joint.

The aim of the present invention is to provide a device for identifying the correct positioning of the femoral component in prosthetic knee surgery that makes it possible to find the correct orientation and, therefore, alignment of the prosthesis to be implanted.

Within this aim, an object of the present invention is to provide a device for identifying the correct positioning of the femoral component in prosthetic knee surgery that can be used during surgery, following the distal resection of the femur.

Another object of the present invention is to provide a device for identifying the correct positioning of the femoral component in prosthetic knee surgery, which makes it possible to considerably simplify the burden of work for the surgeon, by reducing positioning errors.

Another object of the present invention is to provide a device for identifying the correct positioning of the femoral component in prosthetic knee surgery that is highly reliable, easily and practically implemented and low cost.

This aim and these and other objects which will become better apparent hereinafter are achieved by a device for identifying the correct positioning of the femoral component in prosthetic knee surgery, characterized in that it comprises an anatomical template adapted to be applied after distal resection of the femur, said template having a concave upper portion and two lateral portions separated by a cavity, each one of said lateral portions being provided with at least one hole, a stem being insertable in said cavity and fixable therein by way of a fixing element which can slide along said cavity.

Further characteristics and advantages of the invention will become better apparent from the description of a preferred, but not exclusive, embodiment of the device according to the present invention, which is illustrated by way of non-limiting example in the accompanying drawings wherein:

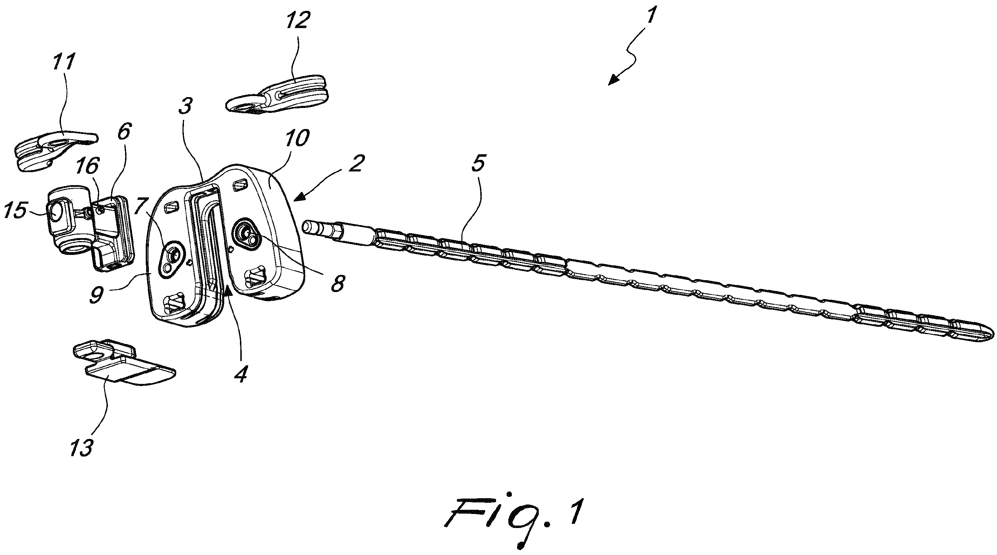

Figure 1 is an exploded perspective view of the device according to the present invention;

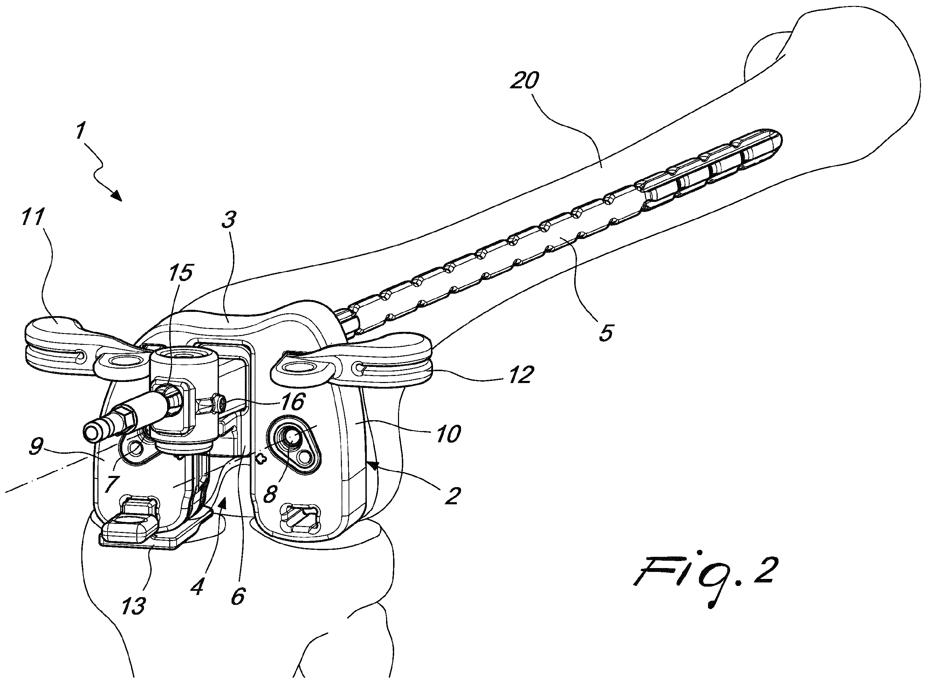

Figure 2 is a perspective view of the device according to the present invention, applied to the femur of a patient;

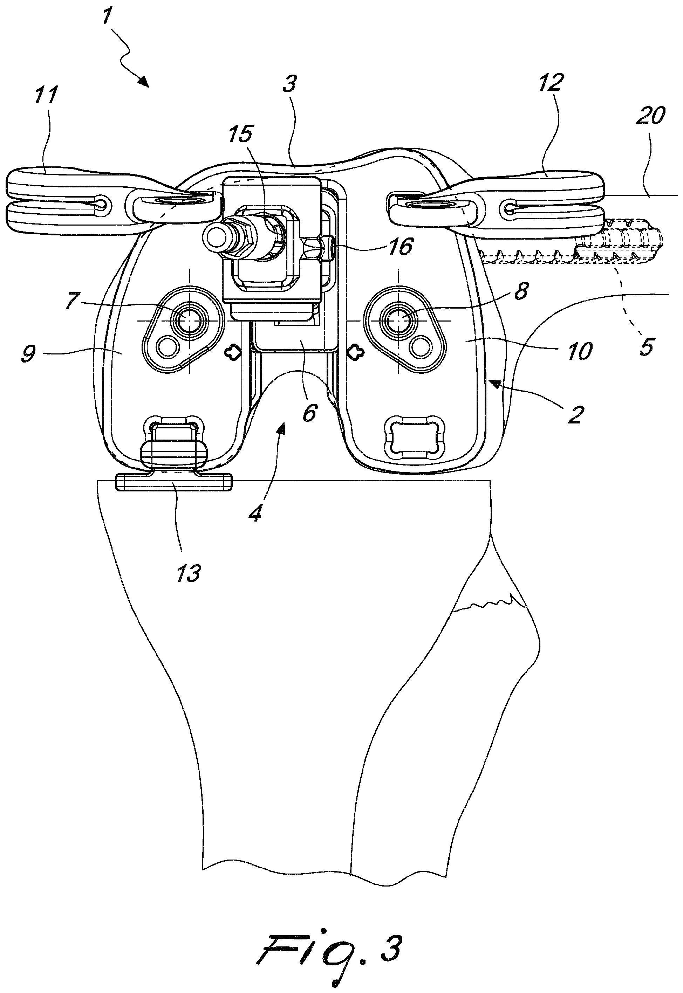

Figure 3 is a perspective front elevation view of the device according to the present invention, applied to the femur of a patient;

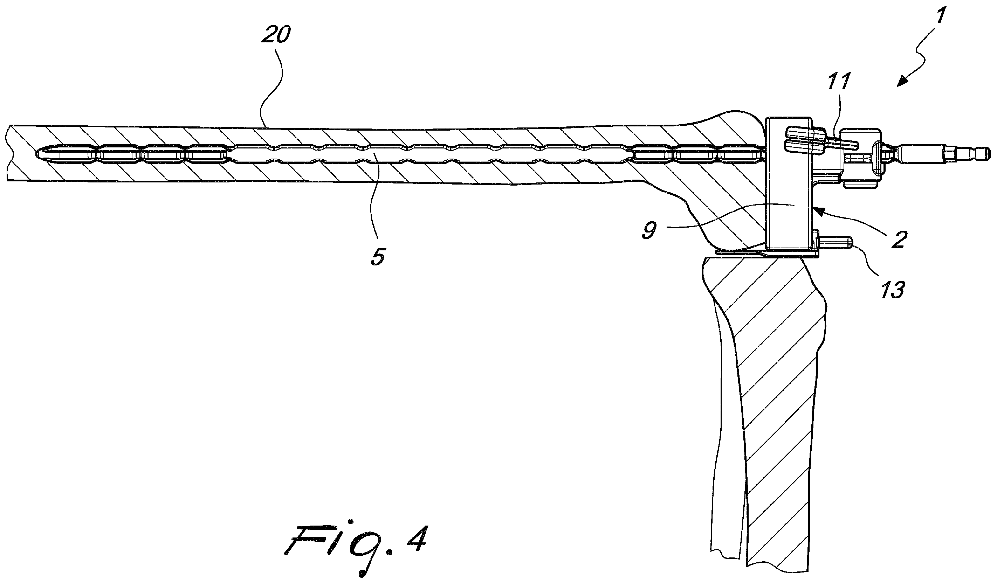

Figure 4 is a cross-sectional side view of the device according to the present invention applied to the femur of a patient;

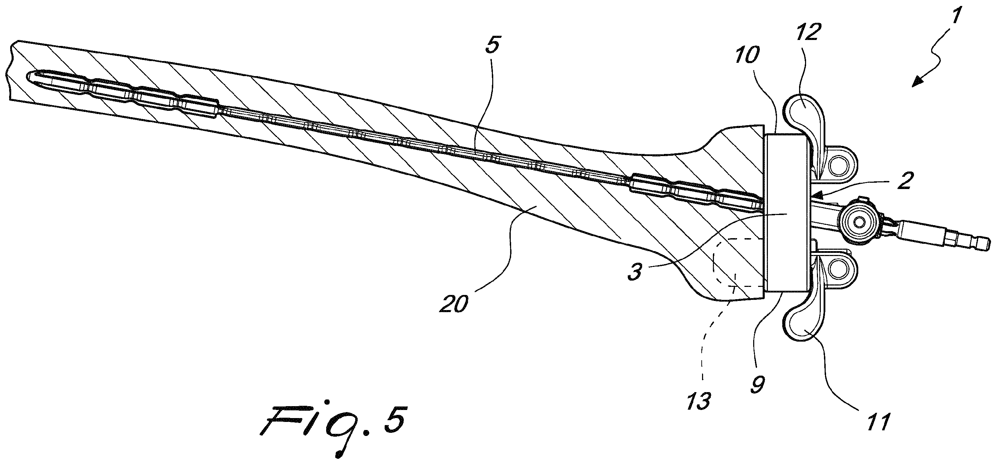

Figure 5 is a transverse cross-sectional plan view from above of the device according to the present invention applied to the femur of a patient.

With reference to the figures, the device according to the present invention, generally designated by the reference numeral 1, comprises a contoured anatomical template 2 with a concave, i.e. hollowed-out, upper portion 3, which reproduces the shape of the patellofemoral prosthetic joint; the femoral template is adapted to be attached to the distal femoral resection surface (it is made in different sizes corresponding to the prosthetic implant) and is oriented on the femoral profile corresponding to the trochlear profile and to the internal femoral condyle using a supporting tab 13 on the posterior medial (otherwise called internal) condyle.

The anatomical template 2 is further provided with a central cavity 4 through which a stem 5 passes which is adapted to be inserted in the medullary canal of the femur 20 of the patient, and the stem 5 is coupled to a fixing element 6 which is accommodated so it can slide in the cavity 4 and fixed therein.

The fixing element is provided with a through hole 15 in which the stem 5 is inserted and locked therein using a grub screw 16.

The anatomical template 2 is further provided conveniently with at least one hole and preferably holes 7 and 8, which are respectively provided at the lateral portions 9 and 10 thereof, and which make it possible to identify a position for fixing the femoral component of the prosthesis that is to be implanted in the patient.

The anatomical template 2 is further provided with a pair of lateral fins **11** and 12, which are respectively fixed at the upper portion of the anatomical template, close to the curved section of the concave upper portion 3.

The lateral fins **11** and 12 serve as reference points so that the surgeon can understand whether the chosen anatomical template 2 is the right size for the femur of the patient.

The template 2 must in fact be joined to the resected part of the femur and be adapted as close as possible to it, so as to substantially reproduce its shape.

The template is provided, as mentioned, with the tab 13 which is adapted to be joined below it, at one of the two lateral portions 9 and 10, in order to provide a position reference for the surgeon.

The femoral component of the prosthesis is then aligned according to the following procedure.

First an anatomical template 2 is chosen that has dimensions that are closest to those of the knee of the patient being operated on, and this template 2 is applied after the distal resection of the femur.

It is necessary first of all to reconstruct the dimensions of the internal femoral condyle where the main flexion-extension movement of the knee occurs, in order to respect, release or tension the internal collateral ligament (depending on the individual case) using a common rear reference with perfect adherence of the template 2 to the resection surface and to the corresponding rear portion of the internal femoral condyle.

The template 2 is positioned by inserting the stem 5 in the medullary canal of the femur and immobilizing the template around that medullary stem using the fixing element 6.

In substance, the stem 5 supplies a fixed and non-modifiable point (dictated by the position of the medullary canal of the femur), around which the template 2 is positioned, with the fixing element being inserted on the stem 5 and protruding from the template 2, from the end opposite to the direction of extension of the femur 20.

The fixing element 6 can slide inside and along the cavity 4 of the template 2 and therefore the template 2 is positioned around such fixing element 6 until its concave upper portion 3 is adapted to and matches up as closely as possible with the shape of the joint portion of the patellofemoral prosthetic implant on the anterior femoral plane of the femur.

The template is then rotated at the level of the trochlear groove, where the positioning of the patella on the flexion-extension axis occurs.

The two lateral fins 11 and 12 can be used as additional confirmation of the dimensions of the femoral component.

When the surgeon is certain of the correct rotatory alignment and of the dimensions of the template 2, then at this point the perforations can be made for the seat and the corresponding cutting guide, and then the surgeon proceeds as routine for a normal prosthetic implant.

The perforations are made through the holes 7 and 8 of the anatomical template 2.

In practice it has been found that the device according to the invention fully achieves the set aim and objects, in that it makes it possible to align the posterior distal femoral articulation line of the femoral component along the functional femoral transverse axis parallel to the tibiofemoral flexion-extension axis.

The kinematic alignment that can be obtained using the device according to the present invention can be defined as a kinematic patellofemoral alignment.

Furthermore, the template described above can be used both for the left knee and the right knee, since it can in fact be rotated through 180 degrees.

The device, thus conceived, is susceptible of numerous modifications and variations, all of which are within the scope of the appended claims.

Moreover, all the details may be substituted by other, technically equivalent elements.

In practice, the materials used, as well as the contingent shapes and dimensions, may be any according to the requirements and to the state of the art.

The disclosures in Italian Patent Application No. 102022000022815 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A device (1) for identifying the correct positioning of the femoral component in prosthetic knee surgery, **characterized in that** it comprises an anatomical template (2) adapted to be applied after distal resection of the femur, said template (2) having a concave upper portion (3) and two lateral portions (9, 10) separated by a cavity (4), each one of said lateral portions being provided with at least one hole (7, 8), a stem (5) being insertable in said cavity (4), a fixing element (6) being also provided, said stem (5) being fixable in said cavity (4) by way of the fixing element (6) which can slide along said cavity (4).

2. The device according to claim 1, **characterized in that** it comprises a pair of fins (11, 12) which are adapted to be fixed to said template (2) in an upper position, laterally to said concave upper portion (3).

3. The device according to claim 1 or 2, **characterized in that** it comprises a positioning indication tab (13) adapted to be applied below one or the other of said lateral portions (9, 10) of said template (2).

4. The device according to one or more of the preceding claims, **characterized in that** said fixing element (6) is provided with a through hole (14) for the passage of said stem (5), said fixing element (6) being configured to be positioned within said cavity (4) of the template (2).

5. The device according to one or more of the preceding claims, **characterized in that** said stem (5) is adapted to be inserted into the medullary canal of the femur (20).

6. The device according to one or more of the preceding claims, **characterized in that** said template (2) has a shape that reproduces the shape of the femur (20) after it has undergone distal resection.

7. The device according to one or more of the preceding claims, **characterized in that** said fins (11, 12) provide a visual reference for the surgeon to assess the chosen size of the template (2) and thus of the femoral component of the prosthesis.

## Patentansprüche

1. Vorrichtung (1) zur Identifizierung der korrekten Positionierung der Femurkomponente bei einer Knieprothesenoperation, **dadurch gekennzeichnet, dass** sie eine anatomische Schablone (2) umfasst, die nach einer distalen Resektion des Femurs angewendet werden kann, wobei die Schablone (2) einen konkaven oberen Abschnitt (3) und zwei seitliche Abschnitte (9, 10) aufweist, die durch einen Hohlraum (4) voneinander getrennt sind, wobei jeder der seitlichen Abschnitte mit mindestens einem Loch (7, 8) versehen ist, wobei ein Schaft (5) in den Hohlraum (4) einführbar ist, wobei außerdem ein Befestigungselement (6) vorgesehen ist, wobei der Schaft (5) in dem Hohlraum (4) mittels des Befestigungselements (6) befestigbar ist, das entlang des Hohlraums (4) gleiten kann.

2. Vorrichtung nach Anspruch 1, **dadurch gekennzeichnet, dass** sie ein Paar Flügel (11, 12) umfasst, die so ausgelegt sind, dass sie an der Schablone (2) in einer oberen Position seitlich des konkaven oberen Abschnitts (3) befestigt werden können.

3. Vorrichtung nach Anspruch 1 oder 2, **dadurch gekennzeichnet, dass** sie eine Positionierungsanzeige-Lasche (13) umfasst, die dazu ausgelegt ist, unterhalb des einen oder anderen der seitlichen Abschnitte (9, 10) der Schablone (2) angebracht zu werden.

4. Vorrichtung nach einem oder mehreren der vorstehenden Ansprüche, **dadurch gekennzeichnet, dass** das Befestigungselement (6) mit einem Durchgangsloch (14) für den Durchgang des Schafts (5) versehen ist, wobei das Befestigungselement (6) so konfiguriert ist, dass es innerhalb des Hohlraums (4) der Schablone (2) positioniert werden kann.

5. Vorrichtung nach einem oder mehreren der vorstehenden Ansprüche, **dadurch gekennzeichnet, dass** der Schaft (5) so ausgelegt ist, dass er in den Markkanal des Femurs (20) eingeführt werden kann.

6. Vorrichtung nach einem oder mehreren der vorstehenden Ansprüche, **dadurch gekennzeichnet, dass** die Schablone (2) eine Form aufweist, die die Form des Femurs (20) nach einer distalen Resektion nachbildet.

7. Vorrichtung nach einem oder mehreren der vorstehenden Ansprüche, **dadurch gekennzeichnet, dass** die Lamellen (11, 12) dem Chirurgen eine visuelle Referenz bieten, um die gewählte Größe der Schablone (2) und damit der Femurkomponente der Prothese zu beurteilen.

## Revendications

1. Dispositif (1) permettant d'identifier le positionnement correct du composant fémoral dans le cadre d'une chirurgie prothétique du genou, **caractérisé en ce qu'**il comprend un gabarit anatomique (2) adapté pour être appliqué après une résection distale du fémur, ledit gabarit (2) comportant une partie supérieure concave (3) et deux parties latérales (9, 10) séparées par une cavité (4), chacune desdites parties latérales étant pourvue d'au moins un trou (7, 8), une tige (5) pouvant être insérée dans ladite cavité (4), un élément de fixation (6) étant également prévu, ladite tige (5) pouvant être fixée dans ladite cavité (4) au moyen de l'élément de fixation (6) qui peut coulisser le long de ladite cavité (4).

2. Le dispositif selon la revendication 1, **caractérisé en ce qu'**il comprend une paire d'ailettes (11, 12) qui sont adaptées pour être fixées audit gabarit (2) dans une position supérieure, latéralement à ladite partie supérieure concave (3).

3. Dispositif selon la revendication 1 ou 2, **caractérisé en ce qu'**il comprend une languette d'indication de positionnement (13) adaptée pour être appliquée sous l'une ou l'autre desdites parties latérales (9, 10) dudit gabarit (2).

4. Dispositif selon une ou plusieurs des revendications précédentes, **caractérisé en ce que** ledit élément de fixation (6) est muni d'un trou traversant (14) pour le passage de ladite tige (5), ledit élément de fixation (6) étant configuré pour être positionné à l'intérieur de ladite cavité (4) du gabarit (2).

5. Dispositif selon une ou plusieurs des revendications précédentes, **caractérisé en ce que** ladite tige (5) est adaptée pour être insérée dans le canal médullaire du fémur (20).

6. Dispositif selon une ou plusieurs des revendications précédentes, **caractérisé en ce que** ledit gabarit (2) a une forme qui reproduit la forme du fémur (20) après qu'il a subi une résection distale.

7. Dispositif selon une ou plusieurs des revendications précédentes, **caractérisé en ce que** lesdites ailettes (11, 12) fournissent une référence visuelle au chirurgien pour évaluer la taille choisie du gabarit (2) et donc du composant fémoral de la prothèse.
